# FASCICULE DE BREVET EUROPEEN

(11) **EP 2 343 036 B1**
(45) Date de publication et mention de la délivrance du brevet: **31.05.2017**
(21) Numéro de dépôt: 10195220.8
(22) Date de dépôt: 15.12.2010
(51) Int. Cl.: A61K 8/06, A61K 8/31, A61K 8/39, A61K 8/92, A61Q 5/12

(54) **Composition cosmétique sous forme de nanoémulsion contenant un alcane linéaire volatil**
Kosmetische Zusammensetzung in Form einer Nanoemulsion, die ein flüchtiges lineares Alkan enthält
Cosmetic composition in the form of a nanoemulsion containing a volatile linear alkane

(30) Priorité: 23.12.2009 FR 0959480
(43) Date de publication de la demande: 13.07.2011
(73) Titulaire: L'Oréal, 75008 Paris (FR)
(72) Inventeur: Desenne, Patricia, 74370, Pringy (FR); Chesneau, Laurent, 92300, Levallois (FR)
(74) Mandataire: Dossmann, Gérard

(56) Documents cités:
- EP-A1- 2 113 240
- EP-A2- 1 813 251
- DE-A1-102008 012 457
- Anonymous: "Paraffine", Wikipédia L'encyclopédie libre , 23 septembre 2010 (2010-09-23), XP002602767, Extrait de l'Internet: URL:http://fr.wikipedia.org/wiki/Paraffine [extrait le 2010-09-23]
- Nicolas Anton, Pascal Gayet, Jean-Pierre Benoit, Patrick Saulnier: "Nano-emulsions and nanocapsules by the PIT method: An investigationon the role of the temperature cycling on the emulsion phase inversion", International Journal of Pharmaceutics , 6 May 2007 (2007-05-06), Retrieved from the Internet: URL:http://ac.els-cdn.com/S037851730700366 3/1-s2.0-S0378517307003663-main.pdf?_tid=4 be1e48a-c314-11e4-b591-00000aab0f6b&acdnat =1425545446_9f5dc6b201966ac886d988f0eae39e e9 [retrieved on 2015-03-04]

## Description

La présente invention se rapporte à une composition cosmétique sous forme de nanoémulsion huile-dans-eau comprenant dans un milieu cosmétiquement acceptable au moins un alcane linéaire volatil, au moins une huile et au moins un lipide amphiphile non-ionique de préférence dans un rapport huile/lipide amphiphile non-ionique particulier, ainsi qu'à son utilisation pour le conditionnement des cheveux.

Dans le domaine de la cosmétique, les nano-émulsions de type huile-dans-eau sont connues pour leurs propriétés conditionnantes comme le démêlage et le lissage des cheveux sans perte de tonicité de la fibre.

Cependant, ce sont des systèmes fluides difficiles à stabiliser sans dégrader le niveau des propriétés conditionnantes.

Par ailleurs, il est connu d'utiliser des solvants volatils dans les produits capillaires de soin rincés ou non rincés. Ces solvants volatils permettent généralement de modifier le rendu sensoriel d'un produit capillaire en allégeant sa texture. Ils peuvent lui conférer également un caractère fondant qui facilite son application sur les cheveux.

Cependant, les solvants volatils, tels que les esters gras liquides, les huiles hydrocarbonées de type isododécane ou isohexadécane, ou les huiles siliconées de type cyclométhicone, n'apportent pas d'épaississement particulier dans une nanoémulsion.

Le document EP 2 113 240 divulgue des compositions cosmétiques pour le soin et/ou le lavage des cheveux comprenant une émulsion directe obtenue par un procédé PIT comprenant une phase lipophile qui comprend une huile minérale et une huile végétale, une phase aqueuse et un tensioactif non-ionique.

Il existe donc un besoin de fournir une composition cosmétique sous forme de nanoémulsion qui présente une consistance épaissie, c'est-à-dire pouvant se présenter sous forme de gel, tout en présentant des propriétés conditionnantes satisfaisantes, en particulier en terme de douceur, de brillance et de légèreté.

La demanderesse a découvert de façon surprenante qu'une composition cosmétique sous forme de nanoémulsion huile-dans-eau comprenant au moins un alcane linéaire volatil, au moins une huile différente du ou desdits alcanes linéaires volatils et au moins un lipide amphiphile non-ionique, permettait de résoudre les problèmes de l'art antérieur et de conduire aux effets recherchés mentionnés précédemment.

Ainsi, l'invention a pour objet une composition cosmétique sous forme de nanoémulsion huile-dans-eau comprenant, dans un milieu cosmétiquement acceptable un ou plusieurs lipides amphiphiles non-ioniques, de 2 à 15 % en poids par rapport au poids total de la composition d'un ou plusieurs alcanes linéaires volatils, un ou plusieurs lipides amphiphiles cationiques et une ou plusieurs huiles différentes du ou desdits alcanes linéaires volatils, le ou les alcanes linéaires volatils comprenant de 7 à 15 atomes de carbone, la taille moyenne en nombre des globules de la nanoémulsion étant comprise entre 1 et 350 nm.

De préférence, le rapport pondéral de la quantité d'huile(s) sur la quantité de lipide(s) amphiphile(s) non-ionique(s) est inférieur ou égal à 7.

Par nanoémulsion, on entend au sens de la présente invention une émulsion huile-dans-eau caractérisée par une taille de globules huileux inférieure à 350 nm, les globules huileux étant stabilisés par une couronne de lipides amphiphiles pouvant éventuellement former une phase cristal liquide de type lamellaire, situés à l'interface huile/phase aqueuse. En l'absence d'agents spécifiques opacifiants, la transparence de ces émulsions provient de la petite taille des globules huileux, petite taille obtenue grâce à l'utilisation d'une énergie mécanique et notamment d'un homogénéisateur haute pression. Les nanoémulsions sont à différencier des microémulsions de par leur structure. En effet, les microémulsions sont des dispersions thermodynamiquement stables constituées de micelles de lipide(s) amphiphile(s) gonflées par de l'huile. De plus, les microémulsions ne nécessitent pas d'énergie mécanique importante pour être réalisées ; elles se forment spontanément par simple mise en contact des constituants.

Les nanoémulsions de l'invention contiennent des globules présentant une taille moyenne en nombre comprise entre 1 et 350 nm, mieux entre 5 et 250 nm, et encore plus préférentiellement entre 10 et 150 nm.

La taille moyenne en nombre des globules peut être déterminée en particulier selon la méthode connue de diffusion quasi-élastique de la lumière. A titre d'appareil utilisable pour cette détermination, on peut citer l'appareil de marque BROOKHAVEN équipé d'un banc optique SX 200 (avec laser à 532 nm), et d'un corrélateur BI 9000. Cet appareil fournit une mesure du diamètre moyen par spectroscopie de corrélation de photon (ou PCS : "Photon Correlation Spectroscopy") qui permet de déterminer le diamètre moyen en nombre à partir du facteur de polydispersité également mesuré par l'appareil.

On peut en outre caractériser la nanoémulsion par mesure de sa turbidité selon la méthode NTU à l'aide d'un turbidimètre de modèle 2100P de HACH Company, à température ambiante. La turbidité des nanoémulsions de l'invention est généralement inférieure à 400 unités NTU, et de préférence comprise entre 10 et 300 unités NTU, mieux de 15 à 250, et plus particulièrement de 20 à 150.

La turbidité des émulsions peut également être mesurée par mesure de la transmittance à 600 nm. La transmittance est généralement supérieure à 85%, de préférence supérieure à 90%, et plus particulièrement supérieure à 94%.

La composition selon l'invention contient un ou plusieurs alcane(s) linéaire(s) volatil(s). Par « un ou plusieurs alcane(s) linéaire(s) volatil(s) », on entend indifféremment « une ou plusieurs huile alcane(s) linéaire(s) volatile(s) ».

Un alcane linéaire volatil convenant à l'invention est liquide à température ambiante (environ 25 °C) et à la pression atmosphérique (760 mm Hg).

Par « alcane linéaire volatil » convenant à l'invention, on entend un alcane linéaire cosmétique, susceptible de s'évaporer au contact de la peau en moins d'une heure, à température ambiante (25°C) et pression atmosphérique (760 mm Hg, c'est-à-dire 101 325 Pa), liquide à température ambiante, ayant notamment une vitesse d'évaporation allant de 0,01 à 15 mg/cm²/min, à température ambiante (25°C) et pression atmosphérique (760 mm Hg).

De façon préférée, le ou les alcanes linéaires volatils convenant à l'invention présentent une vitesse d'évaporation allant de 0,01 à 3,5 mg/cm²/min, à température ambiante (25°C) et pression atmosphérique (760 mm Hg).

De façon préférée, le ou les alcanes linéaires volatils convenant à l'invention présentent une vitesse d'évaporation allant de 0,01 à 1,5 mg/cm²/min, à température ambiante (25°C) et pression atmosphérique (760 mm Hg).

De façon plus préférée, le ou les alcanes linéaires volatils convenant à l'invention présentent une vitesse d'évaporation allant de 0,01 à 0,8 mg/cm²/min, à température ambiante (25°C) et pression atmosphérique (760 mm Hg).

De façon encore préférée, le ou les alcanes linéaires volatils convenant à l'invention présentent une vitesse d'évaporation allant de 0,01 à 0,3 mg/cm²/min, à température ambiante (25°C) et pression atmosphérique (760 mm Hg).

De façon encore préférée, le ou les alcanes linéaires volatils convenant à l'invention présentent une vitesse d'évaporation allant de 0,01 à 0,12 mg/cm²/min, à température ambiante (25°C) et pression atmosphérique (760 mm Hg).

La vitesse d'évaporation d'un alcane volatil conforme à l'invention (et plus généralement d'un solvant volatil) peut être notamment évaluée au moyen du protocole décrit dans WO 06/013413, et plus particulièrement au moyen du protocole décrit ci-après.

On introduit, dans un cristallisoir (diamètre : 7 cm) placé sur une balance se trouvant dans une enceinte d'environ 0,3 m³ régulée en température (25 °C) et en hygrométrie (humidité relative 50 %), 15 g de solvant hydrocarboné volatil.

On laisse le liquide s'évaporer librement, sans l'agiter, en assurant une ventilation par un ventilateur (PAPST-MOTOREN, référence 8550 N, tournant à 2700 tours/minute) disposé en position verticale au-dessus du cristallisoir contenant le solvant hydrocarboné volatil, les pales étant dirigées vers le cristallisoir, à une distance de 20 cm par rapport au fond du cristallisoir.

On mesure à intervalles de temps réguliers la masse de solvant hydrocarboné volatil restante dans le cristallisoir.

On obtient alors le profil d'évaporation du solvant en traçant la courbe de la quantité de produit évaporé (en mg/cm²) en fonction du temps (en min).

Puis on calcule la vitesse d'évaporation qui correspond à la tangente à l'origine de la courbe obtenue. Les vitesses d'évaporation sont exprimées en mg de solvant volatil évaporé par unité de surface (cm²) et par unité de temps (minute).

Selon un mode de réalisation préféré, le ou les alcanes linéaires volatils convenant à l'invention ont une pression de vapeur (appelée également pression de vapeur saturante) non nulle, à température ambiante, en particulier une pression de vapeur allant de 0,3 Pa à 6000 Pa.

De façon préférée, le ou les alcanes linéaires volatils convenant à l'invention ont une pression de vapeur allant de 0,3 à 2000 Pa, à température ambiante (25°C).

De façon préférée, le ou les alcanes linéaires volatils convenant à l'invention ont une pression de vapeur allant de 0,3 à 1000 Pa, à température ambiante (25°C)

De façon plus préférée, le ou les alcanes linéaires volatils convenant à l'invention ont une pression de vapeur allant de 0,4 à 600 Pa, à température ambiante (25°C).

De façon préférée, le ou les alcanes linéaires volatils convenant à l'invention ont une pression de vapeur allant de 1 à 200 Pa, à température ambiante (25°C).

De façon encore préférée, le ou les alcanes linéaires volatils convenant à l'invention ont une pression de vapeur allant de 3 à 60 Pa, à température ambiante (25°C).

Selon un mode de réalisation, le ou les alcanes linéaires volatils convenant à l'invention peuvent présenter un point éclair compris dans l'intervalle variant de 30 à 120 °C, et plus particulièrement de 40 à 100 °C. Le point éclair est en particulier mesuré selon la Norme iso 3679.

Le ou les alcanes linéaires volatils convenant à l'invention sont les alcanes linéaires comprenant de 7 à 15 atomes de carbone.

De façon préférée, le ou les alcanes linéaires volatils convenant à l'invention peuvent être les alcanes linéaires comprenant de 8 à 14 atomes de carbone.

De façon préférée, le ou les alcanes linéaires volatils convenant à l'invention peuvent être les alcanes linéaires comprenant de 9 à 14 atomes de carbone.

De façon préférée, le ou les alcanes linéaires volatils convenant à l'invention peuvent être les alcanes linéaires comprenant de 10 à 14 atomes de carbone.

De façon préférée, le ou les alcanes linéaires volatils convenant à l'invention peuvent être les alcanes linéaires comprenant de 11 à 14 atomes de carbone.

Selon un mode de réalisation avantageux, le ou les alcanes linéaires volatils convenant à l'invention présentent une vitesse d'évaporation, telle que définie plus haut, allant de 0,01 à 3,5 mg/cm²/min, à température ambiante (25°C) et pression atmosphérique (760 mm Hg), et comprennent de 8 à 14 atomes de carbone.

Le ou les alcanes linéaires volatils convenant à l'invention peuvent être avantageusement d'origine végétale.

De préférence, l'alcane linéaire volatil ou le mélange d'alcanes linéaires volatils présent dans la composition selon l'invention comprend au moins un isotope ¹⁴C du carbone (carbone 14), en particulier l'isotope ¹⁴C peut être présent en un ratio ¹⁴C / ¹²C supérieur ou égal à 1.10⁻¹⁶. de préférence supérieur ou égal à 1.10⁻¹⁵, de préférence encore supérieur ou égal 7,5.10⁻¹⁴, et mieux supérieur ou égal 1,5.10⁻¹³. De préférence, le ratio ¹⁴C / ¹²C va de 6.10⁻¹³ à 1,2.10⁻¹² (rapport en nombre d'isotopes).

La quantité d'isotopes ¹⁴C dans l'alcane linéaire volatil ou le mélange d'alcanes linéaires volatils peut être déterminée par des méthodes connues de l'homme du métier telles que la méthode de comptage de Libby, la spectrométrie à scintillation liquide ou encore la spectrométrie de masse à accélération (Accelerator Mass Spectrometry).

Un tel alcane peut être obtenu, directement ou en plusieurs étapes, à partir d'une matière première végétale comme une huile, un beurre, une cire, etc.

A titre d'exemple d'alcanes linéaires volatils convenant à l'invention, on peut mentionner les alcanes décrits dans les demandes de brevets de la société Cognis WO 2007/068371, ou WO2008/155059 (mélanges d'alcanes distincts et différant d'au moins un carbone). Ces alcanes sont obtenus à partir d'alcools gras, eux-mêmes obtenus à partir d'huile de coprah ou de palme.

A titre d'exemple d'alcanes linéaires volatils convenant à l'invention, on peut citer le n-heptane (C7), le n-octane (C8), le n-nonane (C9), le n-décane (C10), le n-undécane (C11), le n-dodécane (C12), le n-tridécane (C13), le n-tétradécane (C14), et leurs mélanges. Selon un mode de réalisation particulier, l'alcane linéaire volatil est choisi parmi le n-nonane, le n-undécane, le n-dodécane, le n-tridécane, le n-tétradécane, et leurs mélanges.

Selon un mode préféré, on peut citer les mélanges de n-undécane (C11) et de n-tridécane (C13) obtenus par exemple aux exemples 1 et 2 de la demande WO2008/155059 de la Société Cognis.

On peut également citer le n-dodécane (C12) et le n-tétradécane (C 14) vendus par Sasol respectivement sous les références PARAFOL 12-97 et PARAFOL 14-97, ainsi que leurs mélanges.

On pourra utiliser l'alcane linéaire volatil seul.

On pourra alternativement ou préférentiellement utiliser un mélange d'au moins deux alcanes linéaires volatils distincts, différant entre eux d'un nombre de carbone n d'au moins 1, en particulier différant entre eux d'un nombre de carbone de 1 ou de 2.

Selon un mode de réalisation, on utilise un mélange d'au moins deux alcanes linéaires volatils distincts comportant de 10 à 14 atomes de carbone et différant entre eux d'un nombre de carbone d'au moins 1. A titre d'exemples, on peut citer notamment les mélanges d'alcanes linéaires volatils C10/C11, C11/C12, ou C12/C13.

Selon un autre mode de réalisation, on utilise un mélange d'au moins deux alcanes linéaires volatils distincts comportant de 10 à 14 atomes de carbone et différant entre eux d'un nombre de carbone d'au moins 2. A titre d'exemples, on peut citer notamment les mélanges d'alcanes linéaires volatils C10/C12, ou C12/C14, pour un nombre de carbone n pair et le mélange C11/C13 pour un nombre de carbone n impair.

Selon un mode préféré, on utilise un mélange d'au moins deux alcanes linéaires volatils comportant de 10 à 14 atomes de carbone distincts et différant entre eux d'un nombre de carbone d'au moins 2, et en particulier un mélange d'alcanes linéaires volatils C11/C13 ou un mélange d'alcanes linéaires volatils C12/C14.

D'autres mélanges associant plus de 2 alcanes linéaires volatils selon l'invention, tels que par exemple un mélange d'au moins 3 alcanes linéaires volatils comportant de 7 à 15 atomes de carbone distincts et différant entre eux d'un nombre de carbone d'au moins 1, font également partie de l'invention, mais les mélanges de 2 alcanes linéaires volatils selon l'invention sont préférés (mélanges binaires), lesdits 2 alcanes linéaires volatils représentant de préférence plus de 95% et mieux plus de 99% en poids de la teneur totale en alcanes linéaires volatils dans le mélange.

Selon un mode particulier de l'invention, dans un mélange d'alcanes linéaires volatils, l'alcane linéaire volatil ayant le nombre de carbone le plus petit est majoritaire dans le mélange.

Selon un autre mode de l'invention, on utilise un mélange d'alcanes linéaires volatils dans lequel l'alcane linéaire volatil ayant le nombre de carbone le plus grand est majoritaire dans le mélange.

A titre d'exemples de mélanges convenant à l'invention, on peut citer notamment les mélanges suivants :
- de 50 à 90% en poids, de préférence de 55 à 80% en poids, préférentiellement encore de 60 à 75% en poids d'alcane linéaire volatil en Cn avec n allant de 7 à 15,
- de 10 à 50% en poids, de préférence de 20 à 45% en poids, de préférence de 24 à 40% en poids, d'alcane linéaire volatil en Cn+x avec x supérieur ou égal à 1, de préférence x=1 ou x=2, avec n+x compris entre 8 et 14,
par rapport au poids total des alcanes dans ledit mélange.

En particulier, ledit mélange d'alcanes linéaires volatiles peut contenir en outre :
- moins de 2% en poids, de préférence moins de 1% en poids d'hydrocarbures ramifiés,
- et/ou moins de 2% en poids, de préférence moins de 1% en poids d'hydrocarbures aromatiques,
- et/ou moins de 2% en poids, de préférence moins de 1% en poids et préférentiellement moins de 0,1% en poids d'hydrocarbures insaturés dans le mélange.

Plus particulièrement, un alcane linéaire volatil convenant à l'invention peut être mis en oeuvre sous la forme d'un mélange n-undécane/n-tri décane.

En particulier, on utilisera un mélange d'alcanes linéaires volatils comprenant :
- de 55 à 80% en poids, de préférence de 60 à 75% en poids d'alcane linéaire volatil en C11 (n-undécane),
- de 20 à 45% en poids, de préférence de 24 à 40% en poids d'alcane linéaire volatil en C13 (n-tridécane),
par rapport au poids total des alcanes dans ledit mélange.

Selon un mode de réalisation particulier, le mélange d'alcanes est un mélange n-undécane/n-tridécane. En particulier un tel mélange peut être obtenu selon l'exemple 1 ou l'exemple 2 du WO 2008/155059.

Selon un autre mode de réalisation particulier, on utilise le n-dodécane vendu sous la référence PARAFOL 12-97 par SASOL.

Selon un autre mode de réalisation particulier, on utilise le n-tétradécane vendu sous la référence PARAFOL 14-97 par SASOL.

Selon encore un autre mode de réalisation, on utilise un mélange de n-dodécane et de n-tétradécane. On peut utiliser en particulier le mélange dodécane/tétradécane dans le rapport pondéral 85/15 commercialisé par la société BIOSYNTHIS sous la référence VEGELIGHT 1214.

Le ou les alcanes linéaires volatils représentent de 2 à 15 % en poids par rapport au poids total de la composition.

Le ou les alcanes linéaires volatils forment, seuls ou avec un ou plusieurs autres composés listés ci-dessous, la phase grasse liquide de la composition.

Comme expliqué précédemment, la composition selon l'invention comprend, outre le ou les alcanes linéaires volatils, une ou plusieurs huiles différentes de ces alcanes linéaires volatils.

Par "huile", on entend tout milieu non aqueux liquide à température ambiante (25°C ± 3°C) et sous pression atmosphérique (760 mm Hg), ayant une solubilité dans l'eau à 25°C inférieure à 1 % en poids, et de préférence inférieure à 0,5% en poids. Les huiles utilisées selon la présente invention comportent de préférence au moins une chaîne comprenant au moins six atomes de carbone ou au moins deux groupements siloxane et de préférence ne contiennent pas de fonction acide carboxylique COOH.

Les huiles utilisables dans la présente invention sont toutes les huiles répondant à cette définition. Elles peuvent être notamment choisies parmi les huiles végétales, les huiles animales, les huiles minérales, les huiles synthétiques, les alcools gras liquides, les huiles siliconées et leurs mélanges.

Comme huile végétale, on peut notamment mentionner l'huile d'amande douce, l'huile d'argan, l'huile d'avocat, l'huile d'arachide, l'huile de camélia, l'huile de carthame, l'huile de calophyllum, l'huile de colza, l'huile de coprah, l'huile de coriandre, l'huile de courge, l'huile de germes de blé, l'huile de jojoba ou cire liquide de jojoba, l'huile de lin, l'huile de macadamia, l'huile de germes de maïs, l'huile de noisette, l'huile de noix, l'huile de vernonia, l'huile de noyau d'abricot, l'huile d'olive, l'huile d'onagre, l'huile de palme, l'huile de passiflore, l'huile de pépins de raisin, l'huile de rosier, l'huile de ricin, l'huile de seigle, l'huile de sésame, l'huile de son de riz, l'huile de soja et l'huile de tournesol.

Comme huile animale, on peut notamment citer le perhydrosqualène.

Comme huile minérale, on peut utiliser une huile de paraffine, une huile de vaseline.

Comme huile synthétique, on peut notamment utiliser les esters gras, les éthers gras, le squalane, les poly(α-oléfines) comme par exemple les polydécènes et les polyisobutènes, les huiles végétales transestérifiées et les huiles halogénées et notamment fluorées.

Comme huile végétale transestérifiée, on peut utiliser l'huile d'olive transestérifée avec de l'hexanol, la cire de jojoba transestérifiée avec de l'éthanol.

Comme huiles fluorées, on peut citer le perfluorométhylcyclopentane et le perfluoro-1,3-diméthylcyclohexane, par exemple vendus sous les dénominations de « FLUTEC PC1® » et « FLUTEC PC3® » par la Société BNFL Fluorochemicals ; le perfluoro-1,2-diméthylcyclobutane ; les perfluoralcanes tels que le dodécafluoropentane et le tétradécafluorohexane, par exemple vendus sous les dénominations de « PF 5050® » et « PF 5060® » par la Société 3M, ou encore le bromoperfluorooctyle par exemple vendu sous la dénomination « FORALKYL® » par la Société Atochem ; le nanofluorométhoxybutane par exemple vendu sous la dénomination « MSX 4518® » par la Société 3M et le nanofluoroéthoxyisobutane ; les dérivés de perfluoromorpholine, tels que la 4-trifluorométhyl perfluoromorpholine par exemple vendue sous la dénomination « PF 5052® » par la Société 3M ; les huiles fluorées partiellement hydrocarbonées comme celles décrites dans le document JP-A-2 295912.

Les esters gras utilisables comme huiles synthétiques peuvent être des esters de monoalcools ou de polyols avec des mono ou des polyacides, le nombre total d'atomes de carbone de l'ester étant supérieur ou égal à 10.

De préférence, au moins un des alcools et/ou des acides comporte au moins une chaîne de plus de 7 atomes de carbone.

Comme esters gras, sont plus préférentiellement employés les composés de formule RₐCOOR_{b} dans laquelle Rₐ représente le reste d'un acide supérieur linéaire ou ramifié, hydroxylé ou non, saturé ou non, comportant de 4 à 29 atomes de carbone et R_{b} représente une chaîne hydrocarbonée linéaire ou ramifiée, saturée ou non contenant de 3 à 30 atomes de carbone, le nombre total d'atomes de carbone de l'ester étant supérieur à 10. A titre d'exemples, on peut notamment citer l'huile de purcellin (octanoate de stéaryle), le myristate d'isopropyle, le palmitate d'isopropyle, le stéarate de butyle, le laurate d'hexyle, l'isononanoate d'isononyle, le palmitate de 2-éthylhexyle, le laurate de 2-hexyldécyle, le palmitate de 2-octyldécyle, le myristate de 2-octyldodécyle, le néopentanoate d'isostéaryle ou le néopentanoate de tridécyle.

Les éthers gras utilisables comme huiles synthétiques sont notamment les composés de formule RₐOR_{b} dans laquelle Rₐ et R_{b} ont les significations données ci-dessus, le nombre total d'atomes de carbone de l'éther étant supérieur à 10.

Les alcools gras utilisables comme huiles sont notamment les alcools gras liquides ayant de 8 à 26 atomes de carbone, comme par exemple l'octyldodécanol, le 2-butyloctanol, l'alcool oléique, l'alcool linoléique ou l'alcool linolénique.

Comme huile siliconée, on peut citer les polyorganosiloxanes, tels que notamment définis dans l'ouvrage de Walter NOLL "Chemistry and Technology of Silicones" (1968) Academic Press. Ils peuvent être volatiles ou non volatiles.

Les polyorganosiloxanes volatiles peuvent être choisis parmi ceux possédant un point d'ébullition compris entre 60° C et 260° C, et plus particulièrement encore parmi :
(i) les silicones cycliques comportant de 3 à 7 atomes de silicium et, de préférence, 4 à 5. Il s'agit, par exemple, de l'octaméthylcyclotétra-siloxane commercialisé notamment sous le nom de "VOLATILE SILICONE 7207" par UNION CARBIDE ou "SILBIONE 70045 V 2" par RHODIA, le décaméthylcyclopentasiloxane commercialisé sous le nom de "VOLATILE SILICONE 7158" par UNION CARBIDE, "SILBIONE 70045 V 5" par RHODIA, ainsi que leurs mélanges.
   On peut également citer les cyclocopolymères du type diméthylsiloxane/méthylalkylsiloxane, tel que la "SILICONE VOLATILE FZ 3109" commercialisée par la société UNION CARBIDE, de structure chimique : avec 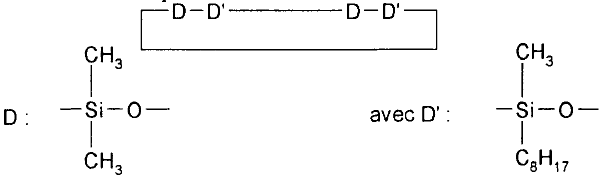
   On peut également citer les mélanges de silicones cycliques avec des composés organiques dérivés du silicium, tels que le mélange d'octaméthylcyclotétrasiloxane et de tétratriméthylsilylpentaérythritol (50/50) et le mélange d'octaméthylcyclotétrasiloxane et d'oxy-1,1'-(hexa-2,2,2',2',3,3'-triméthylsilyloxy) bis-néopentane ;
(ii) les silicones volatiles linéaires ayant 2 à 9 atomes de silicium et possédant une viscosité inférieure ou égale à 5.10⁻⁶m²/s à 25 °C. Il s'agit, par exemple, du décaméthyltétrasiloxane commercialisé notamment sous la dénomination "SH 200" par la société TORAY SILICONE. Des silicones entrant dans cette classe sont également décrites dans l'article publié dans Cosmetics and toiletries, Vol. 91, Jan. 76, p. 27-32 - TODD & BYERS "Volatile Silicone fluids for cosmetics".

Les polyorganosiloxanes non volatiles peuvent être choisis parmi les polyalkylsiloxanes, les polyarylsiloxanes, les polyalkylarylsiloxanes, les polyorganosiloxanes modifiés par des groupements organofonctionnels, les copolymères blocs linéaires polysiloxane(A)-polyoxyalkylène(B) de type (A-B)ₙ avec n >3 ; les polymères siliconés greffés, à squelette organique non siliconé, constitués d'une chaîne principale organique formée à partir de monomères organiques ne comportant pas de silicone, sur laquelle se trouve greffé, à l'intérieur de ladite chaîne ainsi qu'éventuellement à l'une au moins de ses extrémités, au moins un macromonomère polysiloxane ; les polymères siliconés greffés, à squelette polysiloxanique greffé par des monomères organiques non siliconés, comprenant une chaîne principale de polysiloxane sur laquelle se trouve greffé, à l'intérieur de ladite chaîne ainsi qu'éventuellement à l'une au moins de ses extrémités, au moins un macromonomère organique ne comportant pas de silicone ; ainsi que leurs mélanges.

A titre d'exemples de polyalkylsiloxanes, on peut notamment citer les polydiméthylsiloxanes à groupements terminaux triméthylsilyle ayant une viscosité de 5.10⁻⁶ à 2,5 m²/s à 25°C et de préférence 1.10⁻⁵ à 1 m²/s. La viscosité des silicones est par exemple mesurée à 25°C selon la norme ASTM 445 Appendice C.

Parmi ces polyalkylsiloxanes, on peut citer à titre non limitatif les produits commerciaux suivants :
- les huiles SILBIONE des séries 47 et 70 047 ou les huiles MIRASIL commercialisées par RHONE POULENC telles que par exemple l'huile 70 047 V 500 000 ;
- les huiles de la série MIRASIL commercialisées par la société RHONE POULENC :
- les huiles de la série 200 de la société DOW CORNING telles que plus particulièrement la DC200 de viscosité 60 000 cSt ;
- les huiles VISCASIL de GENERAL ELECTRIC et certaines huiles des séries SF (SF 96, SF 18) de GENERAL ELECTRIC.

On peut également citer les polydiméthylsiloxanes à groupements terminaux diméthylsilanol (Dimethiconol selon la dénomination CTFA) tels que les huiles de la série 48 de la société RHONE POULENC.

Les polyalkylarylsiloxanes peuvent être notamment choisis parmi les polydiméthyl-méthylphénylsiloxanes, les polydiméthyl-diphénylsiloxanes linéaires et/ou ramifiés de viscosité de 1.10⁻⁵ à 5.10⁻² m²/s à 25°C.

Parmi ces polyalkylarylsiloxanes, on peut citer à titre d'exemple les produits commercialisés sous les dénominations suivantes :
- les huiles SILBIONE de la série 70 641 de RHONE POULENC;
- les huiles des séries RHODORSIL 70 633 et 763 de RHONE POULENC ;
- l'huile DOW CORNING 556 COSMETIC GRAD FLUID de DOW CORNING ;
- certaines huiles des séries SF de GENERAL ELECTRIC telles que SF 1023, SF 1154, SF 1250, SF 1265.

Comme huile siliconée, on peut également utiliser des silicones organomodifiées, qui sont des silicones telles que définies précédemment et comportant dans leur structure un ou plusieurs groupements organofonctionnels fixés par l'intermédiaire d'un groupe hydrocarboné.

Parmi les silicones organomodifiées utilisables selon l'invention, on peut citer les polyorganosiloxanes comportant :
- des groupements aminés substitués ou non
   - des groupements thiols ;
   - des groupements alcoxylés ;
   - des groupements hydroxylés ;
- des groupements acyloxyalkyle ;
- des groupements hydroxyacylamino.

Les huiles particulièrement préférées dans la composition selon l'invention sont notamment les huiles végétales, et plus particulièrement l'huile d'olive et la cire liquide de jojoba ; les huiles siliconées et plus particulièrement les polydiméthylsiloxanes linéaires ou cycliques; et les huiles minérales telle que notamment l'huile de vaseline.

L'huile ou les huiles sont avantageusement présentes dans la composition selon l'invention en une quantité allant de 1 à 40 % en poids, de préférence de 2 à 15 % en poids, par rapport au poids total de la composition.

Comme expliqué précédemment, la composition selon l'invention comprend un ou plusieurs lipides amphiphiles (ou tensioactifs) non-ioniques.

Dans la présente demande, on utilisera indifféremment les termes « lipide amphiphile » et « tensioactif ».

Les lipides amphiphiles non-ioniques de l'invention sont préférentiellement choisis parmi :
1/- les tensioactifs siliconés,
2/- les lipides amphiphiles fluides à température inférieure ou égale à 45°C choisis parmi les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée,
3/- les esters mixtes d'acide gras en C₈-C₂₂ ou d'alcool gras en C₈-C₂₂, d'acide carboxylique et de glycérol,
4/- les esters d'acide gras et de sucre et les éthers d'alcool gras et de sucre,
5/- les tensioactifs solides à une température inférieure ou égale à 45°C, choisis parmi les esters gras de glycérol, les esters gras de sorbitan et les esters gras de sorbitan oxyéthylénés, les éthers gras éthoxylés et les esters gras éthoxylés, et
6/- les copolymères blocs d'oxyde d'éthylène (A) et d'oxyde de propylène (B).

1/ Les tensioactifs siliconés utilisables selon l'invention sont des composés siliconés comportant au moins une chaîne oxyéthylénée-OCH₂CH₂-et/ou oxypropylénée -OCH₂CH₂CH₂-. Comme tensioactifs siliconés pouvant être utilisés selon la présente invention, on peut citer ceux décrits dans les documents US-A-5364633 et US-A-5411744.
   De préférence, le tensioactif siliconé utilisé selon la présente invention est un composé de formule (I) : 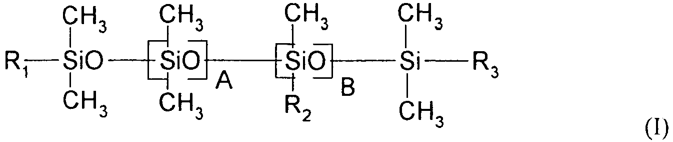 dans laquelle :
   R₁, R₂, R₃, indépendamment les uns des autres, représentent un radical alkyle en C₁-C₆ ou un radical -(CH₂)ₓ-(OCH₂CH₂)_{y}-(OCH₂CH₂CH₂)_{z}-OR₄, au moins un radical R₁, R₂ ou R₃ n'étant pas un radical alkyle ; R₄ étant un hydrogène, un radical alkyle C1-C6 ou un radical acyle ;
   A est un nombre entier allant de 0 à 200 ;
   B est un nombre entier allant de 0 à 50 ; à la condition que A et B ne soient pas égaux à zéro en même temps ;
   x est un nombre entier allant de 1 à 6 ;
   y est un nombre entier allant de 1 à 30 ;
   z est un nombre entier allant de 0 à 5.

   Selon un mode de réalisation préféré de l'invention, dans le composé de formule (I), le radical alkyle est un radical méthyle, x est un nombre entier allant de 2 à 6 et y est un nombre entier allant de 4 à 30.
   On peut citer, à titre d'exemple de tensioactifs siliconés de formule (I), les composés de formule (II) : 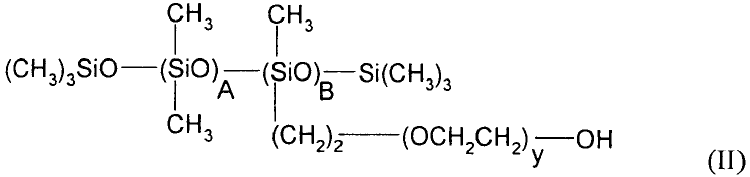 dans laquelle A est un nombre entier allant de 20 à 105, B est un nombre entier allant de 2 à 10 et y est un nombre entier allant de 10 à 20.
   On peut également citer à titre d'exemple de tensioactifs siliconés de formule (I), les composés de formule (III) :

   H-(OCH₂CH₂)_{y}-(CH₂)₃-[(CH₃)₂SiO]_{A'}-(CH₂)₃-(OCH₂CH₁)_{y} -OH (III)

   dans laquelle A' et y sont des nombres entiers allant de 10 à 20.
   On peut utiliser comme composés de l'invention ceux commercialisés par la société Dow Corning sous les dénominations DC 5329, DC 7439-146, DC 2-5695 et Q4-3667. Les composés DC 5329, DC 7439-146, DC 2-5695 sont des composés de formule (II) où respectivement A est 22, B est 2 et y est 12 ; A est 103, B est 10 et y est 12 ; A est 27, B est 3 et y est 12.
   Le composé Q4-3667 est un composé de formule (III) où A est 15 et y est 13.
2/ les lipides amphiphiles fluides à température inférieure ou égale à 45°C sont notamment :
   - l'isostéarate de polyéthylèneglycol de poids moléculaire 400, vendu sous la dénomination PEG 400 par la société UNICHEMA ;
   - l'isostéarate de diglycéryle, vendu par la société SOLVAY ;
   - le laurate de glycérol comportant 2 unités de glycérol, vendu par la société SOLVAY ;
   - L'oléate de sorbitane, vendu sous la dénomination SPAN 80 par la société ICI ;
   - l'isostéarate de sorbitane, vendu sous la dénomination NIKKOL SI 10R par la société NIKKO ;
   - le cocoate d'α-butylglucoside ou le caprate d'α-butylglucoside commercialisés par la société ULICE.
3/ Les esters mixtes d'acide gras en C₈-C₂₂ ou d'alcool gras en C₈-C₂₂, d'acide carboxylique et de glycérol, utilisables comme lipides amphiphiles non-ioniques dans la composition selon l'invention peuvent être choisis notamment dans le groupe comprenant les esters mixtes d'acide gras ou d'alcool gras ayant une chaîne alkyle comportant de 8 à 22 atomes de carbone, et d'alpha-hydroxyacide et/ou d'acide succinique, avec la glycérine. L'alpha-hydroxyacide peut être par exemple l'acide citrique, l'acide lactique, l'acide glycolique, l'acide malique et leurs mélanges.
   La chaîne alkyle des acides ou alcools gras dont dérivent les esters mixtes utilisables dans la composition de l'invention peut être linéaire ou ramifiée, saturée ou non saturée. Il peut s'agir notamment de chaînes stéarate, isostéarate, linoléate, oléate, béhénate, arachidonate, palmitate, myristate, laurate, caprate, isostéaryle, stéaryle, linoléyle, oléyle, béhényle, myristyle, lauryle, capryle et leurs mélanges.
   On peut citer, à titre d'exemple d'esters mixtes utilisables dans la composition de l'invention, l'ester mixte de glycérine et du mélange d'acides citrique, lactique, linoléique et oléique (nom CTFA : Glyceryl citrate/lactate/linoleate/oleate) commercialisé par la société Hüls sous la dénomination Imwitor 375 ; l'ester mixte d'acide succinique et d'alcool isostéarylique avec la glycérine (nom CTFA Isostéaryl diglycéryl succinate) commercialisé par la société Hüls sous la dénomination Imwitor 780 K ; l'ester mixte d'acide citrique et d'acide stéarique avec la glycérine (nom CTFA : Glyceryl stearate citrate) commercialisé par la société Hüls sous la dénomination Imwitor 370 ; l'ester mixte d'acide lactique et d'acide stéarique avec la glycérine
   (nom CTFA : Glyceryl stearate lactate) commercialisé par la société Danisco sous la dénomination Lactodan B30 ou Rylo LA30.
4/ Les esters d'acide gras et de sucre, utilisables comme lipides amphiphiles non-ioniques dans la composition selon l'invention sont de préférence solides à une température inférieure ou égale à 45°C et peuvent être choisis notamment dans le groupe comprenant les esters ou les mélanges d'esters d'acide gras en C₈-C₂₂ et de sucrose, de maltose, de glucose ou de fructose, et les esters ou les mélanges d'esters d'acide gras en C₁₄-C₂₂ et de méthylglucose.

Les acides gras en C₈-C₁₂ ou en C₁₄-C₂₂ formant le motif gras des esters utilisables dans la composition de l'invention comportent une chaîne alkyle linéaire saturée ou non saturée, comportant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.

On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta F50, F70, F110, F160 ayant respectivement un HLB (Hydrophilic Lipophilic Balance) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450. On peut citer aussi les monoesters de glucose ou de maltose tels que l'o-hexadécanoyle-6-D-glucoside de méthyle et l'o-hexadécanoyle-6-D-maltoside.

Les éthers d'alcool gras et de sucre, utilisables comme lipides amphiphiles non-ioniques dans la composition selon l'invention sont solides à une température inférieure ou égale à 45°C et peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.

Les alcools gras en C₈-C₂₂ ou en C₁₄-C₂₂ formant le motif gras des éthers utilisables dans la composition de l'invention comportent une chaîne alkyle linéaire saturée ou non saturée, comportant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle, et leurs mélanges tels que cétéaryle.

A titre d'exemple d'éthers d'alcool gras et de sucre, on peut citer les alkylpolyglucosides tels que le décyl glucoside et le lauryl glucoside commercialisés par exemple par la société Henkel sous les dénominations respectives Plantaren 2000 et Plantaren 1200, le cétostéaryl glucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidyl glucoside, par exemple sous la forme du mélange d'alcools arachidique et béhénique et d'arachidyl glucoside commercialisé sous la dénomination Montanov 202 par la société Seppic.

On utilise plus particulièrement comme ester d'acide gras et de sucre et comme éther d'alcool gras et de sucre le monostéarate de sucrose, le distéarate de sucrose, le tristéarate de sucrose et leurs mélanges, le distéarate de méthyl glucose et de polyglycérol-3 et les alkylpolyglucosides.
5/ Les esters gras de glycérol, utilisables comme lipides amphiphiles non-ioniques dans la composition selon l'invention solides à une température inférieure ou égale à 45°C peuvent être choisis notamment dans le groupe comprenant les esters formés d'au moins un acide comportant une chaîne alkyle linéaire saturée, ayant de 16 à 22 atomes de carbone, et de 1 à 10 motifs glycérol. On peut utiliser un ou plusieurs de ces esters gras de glycérol dans la composition de l'invention.

Ces esters peuvent être notamment choisis parmi les stéarates, béhénates, arachidates, palmitates et leurs mélanges. On utilise de préférence des stéarates et palmitates.

On peut citer à titre d'exemple d'ester gras de glycérol utilisable dans la composition de l'invention, les monostéarate, distéarate, tristéarate et pentastéarate de décaglycérol (noms CTFA : Polyglyceryl-10 stearate, Polyglyceryl-10 distearate, Polyglyceryl-10 tristearate, Polyglyceryl-10 pentastearate) tels que les produits vendus sous les dénominations respectives Nikkol Decaglyn 1-S, 2-S, 3-S et 5-S par la société Nikko, et le monostéarate de diglycérol (nom CTFA : Polyglyceryl-2 stearate) tel que le produit vendu par la société Nikko sous la dénomination Nikkol DGMS.

Les esters gras de sorbitan, utilisables comme lipides amphiphiles non-ioniques dans la composition selon l'invention sont solides à une température inférieure ou égale à 45°C et sont choisis dans le groupe comprenant les esters d'acide gras en C₁₆-C₂₂ et de sorbitan et les esters d'acide gras en C₁₆-C₂₂ et de sorbitan oxyéthylénés. Ils sont formés d'au moins un acide gras comportant au moins une chaîne alkyle linéaire saturée, ayant respectivement de 16 à 22 atomes de carbone, et de sorbitol ou de sorbitol éthoxylé. Les esters oxyéthylénés comportent généralement de 1 à 100 unités d'éthylène glycol et de préférence de 2 à 40 unités d'oxyde d'éthylène (OE).

Ces esters peuvent être notamment choisis parmi les stéarates, béhénates, arachidates, palmitates, et leurs mélanges. On utilise de préférence des stéarates et palmitates.

On peut citer à titre d'exemple d'ester gras de sorbitan utilisable dans la composition de l'invention, le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, le tristéarate de sorbitan 20 OE (nom CTFA : Polysorbate 65) vendu par la société ICI sous la dénomination Tween 65.

Les éthers gras éthoxylés solides à une température inférieure ou égale à 45°C, utilisables comme lipides amphiphiles non-ioniques dans la composition selon l'invention sont de préférence des éthers formés de 1 à 100 unités d'oxyde d'éthylène et d'au moins une chaîne d'alcool gras ayant de 16 à 22 atomes de carbone. La chaîne grasse des éthers peut être notamment choisie parmi les motifs béhényle, arachidyle, stéaryle, cétyle, et leurs mélanges tels que cétéaryle. A titre d'exemple d'éthers gras éthoxylés, on peut citer les éthers d'alcool béhénique comprenant 5, 10, 20 et 30 unités d'oxyde d'éthylène (noms CTFA : Beheneth-5, Beheneth-10, Beheneth-20, Beheneth-30), tels que les produits commercialisés sous les dénominations Nikkol BB5, BB10, BB20, BB30 par la société Nikko, et l'éther d'alcool stéarylique comprenant 2 unités d'oxyde d'éthylène (nom CTFA : Steareth-2), tel que le produit commercialisé sous la dénomination Brij 72 par la société ICI.

Les esters gras éthoxylés solides à une température inférieure ou égale à 45°C, utilisables comme lipides amphiphiles non-ioniques dans la composition selon l'invention sont de préférence des esters formés de 1 à 100 unités d'oxyde d'éthylène et d'au moins une chaîne d'acide gras comportant de 16 à 22 atomes de carbone. La chaîne grasse des esters peut être notamment choisie parmi les motifs stéarate, béhénate, arachidate, palmitate, et leurs mélanges. A titre d'exemple d'esters gras éthoxylés, on peut citer l'ester d'acide stéarique comprenant 40 unités d'oxyde d'éthylène, tel que le produit commercialisé sous la dénomination Myrj 52 (nom CTFA : PEG-40 stearate) par la société ICI ainsi que l'ester d'acide béhénique comprenant 8 unités d'oxyde d'éthylène (nom CTFA PEG-8 behenate), tel que le produit commercialisé sous la dénomination Compritol HD5 ATO par la société Gattefosse.
6/ Les copolymères blocs d'oxyde d'éthylène (A) et d'oxyde de propylène (B), utilisables comme lipides amphiphiles non-ioniques dans la composition selon l'invention peuvent être choisis notamment parmi les copolymères blocs de formule (IV) :

   HO(C₂H₄O)x(C₃H₆O)y(C₂H₄O)zH (IV)

   dans laquelle x, y et z sont des nombres entiers tels que x+z va de 2 à 100 et y va de 14 à 60, et leurs mélanges, et plus particulièrement parmi les copolymères blocs de formule (I) ayant un HLB allant de 2 à 16.

Ces copolymères blocs peuvent être notamment choisis parmi les poloxamers et notamment parmi le Poloxamer 231 tel que le produit commercialisé par la société ICI sous la dénomination Pluronic L81 de formule (I) avec x=z=6, y=39 (HLB 2) ; le Poloxamer 282 tel que le produit commercialisé par la société ICI sous la dénomination Pluronic L92 de formule (I) avec x=z=10, y=47 (HLB 6) ; et le Poloxamer 124 tel que le produit commercialisé par la société ICI sous la dénomination Pluronic L44 de formule (I) avec x=z=11, y=21 (HLB 16).

Parmi les lipides amphiphiles non ioniques, on préfère utiliser :
- l'isostéarate de polyéthylèneglycol (8 moles d'oxyde d'éthylène),
- l'isostéarate de diglycéryle,
- le monolaurate et le monostéarate de polyglycérol comportant 10 unités de glycérol,
- l'oléate de sorbitane, et
- l'isostéarate de sorbitane.

Le ou les lipides amphiphiles non-ioniques représentent généralement de 0,1 à 30%, de préférence de 0,5 à 20%, mieux de 1 à 10% en poids du poids total de la composition.

Selon leur caractère plus hydrophile ou plus lipophile, le ou les lipides amphiphiles non ioniques peuvent être introduits dans la phase aqueuse ou dans la phase huileuse de la nanoémulsion.

Le rapport en poids de la quantité d'huile(s) sur la quantité de lipide(s) amphiphile(s) non-ionique(s) est de préférence inférieur ou égal à 7, mieux compris entre 0,5 et 7, et encore plus préférentiellement inférieur ou égal à 5, mieux compris entre 0,5 et 5. On entend ici par quantité d'huile(s) la quantité totale de l'huile ou des huiles présentes dans la composition, sans inclure la quantité de lipide amphiphile non-ionique ni la quantité d'alcanes linéaires volatils.

La composition selon l'invention comprend un ou plusieurs lipides amphiphiles cationiques. Selon une forme particulière de l'invention, la composition selon l'invention peut également comprendre un ou plusieurs lipides amphiphiles anioniques. Le ou les lipides amphiphiles cationiques et/ou anioniques ne sont pas inclus dans la quantité d'huile pour l'évaluation du rapport pondéral de la quantité d'huile(s) sur la quantité de lipide(s) amphiphile(s) non-ionique(s).

Le ou les lipides amphiphiles cationiques, présents dans la composition de l'invention, sont choisis, de préférence, dans le groupe formé par les sels d'ammonium quaternaire, les amines grasses et leurs sels.

Les sels d'ammonium quaternaires sont par exemple :
- ceux qui présentent la formule générale (V) suivante : 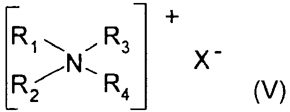 dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle, au moins un des radicaux R₁ à R₄ ayant de 8 à 30 atomes de carbone et de préférence de 12 à 24. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,
- les sels d'ammonium quaternaire de l'imidazolinium, comme par exemple celui de formule (VI) suivante : 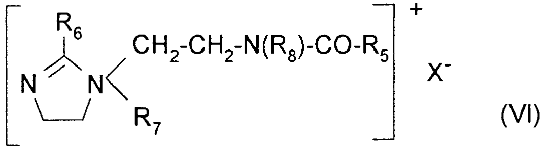 dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁-C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination « REWOQUAT W 75» par la société REWO,
- les sels de diammonium quaternaire de formule (VII) : 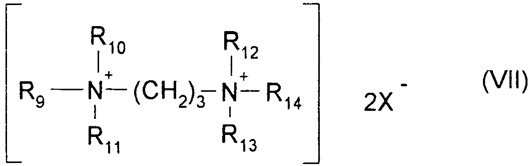 dans laquelle R₉ désigne un radical aliphatique comportant environ de 16 à 30 atomes de carbone, R₁₀, R₁₁, R₁₂, R₁₃ et R₁₄, identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester.

Les sels d'ammonium quaternaire contenant au moins une fonction ester utilisables selon l'invention sont par exemple ceux de formule (VIII) suivante : 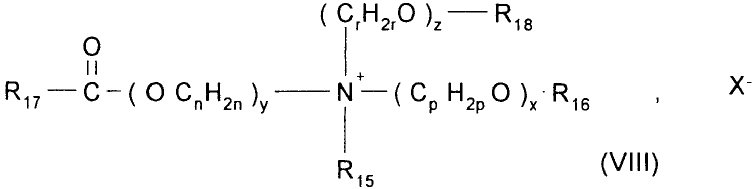 dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
- le radical 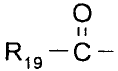
   - les radicaux R₂₀ hydrocarbonés en C₁-C₂₂ linéaires ou ramifiés, saturés ou insaturés,
   - l'atome d'hydrogène,
- R₁₈ est choisi parmi :
   - le radical 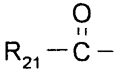
   - les radicaux R₂₂ hydrocarbonés en C₁-C₆ linéaires ou ramifiés, saturés ou insaturés,
   - l'atome d'hydrogène,
   - R₁₇, R₁₉ et R₂₁, identiques ou différents, sont choisis parmi les radicaux hydrocarbonés en C₇-C₂₁, linéaires ou ramifiés, saturés ou insaturés ;
   - n, p et r, identiques ou différents, sont des entiers valant de 2 à 6 ;
   - y est un entier valant de 1 à 10 ;
   - x et z, identiques ou différents, sont des entiers valant de 0 à 10 ;
   - X⁻ est un anion simple ou complexe, organique ou inorganique ;
   sous réserve que la somme x + y + z vaut de 1 à 15 , que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁-C₂₁, linéaires ou ramifiés, saturés ou insaturés.

De préférence, x et z, identiques ou différents, valent 0 ou 1.

Avantageusement, y est égal à 1.

De préférence, n, p et r, identiques ou différents, valent 2 ou 3 et encore plus particulièrement sont égaux à 2.

L'anion est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester.

L'anion X⁻ est encore plus particulièrement le chlorure ou le méthylsulfate.

On utilise plus particulièrement les sels d'ammonium de formule (VIII) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 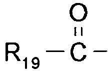
   - les radicaux méthyle, éthyle ou hydrocarbonés en C₁₄-C₂₂
   - l'atome d'hydrogène ;
- R₁₈ est choisi parmi :
   - le radical 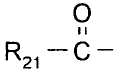
   - l'atome d'hydrogène ;
R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.

Avantageusement, les radicaux hydrocarbonés sont linéaires.

On peut citer par exemple les composés de formule (VIII) tels que les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents.

Ces produits sont obtenus par exemple par estérification directe de la triéthanolamine, de la triisopropanolamine, d'alkyldiéthanolamine ou d'alkyldiisopropanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations DEHYQUART par la société HENKEL, STEPANQUAT par la société STEPAN, NOXAMIUM par la société CECA, REWOQUAT WE 18 par la société REWO-WITCO.

La composition selon l'invention contient de préférence un sel d'ammonium quaternaire de formule (V).

Parmi les sels d'ammonium quaternaire de formule (V) on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «CERAPHYL 70» par la société VAN DYK. Selon l'invention, le chlorure de béhényltriméthylammonium et le chlorure de cétyl triméthyl ammonium sont les sels d'ammonium quaternaire les plus particulièrement préférés. La composition selon l'invention peut également comprendre un ou plusieurs lipides amphiphiles anioniques. Le ou les lipides amphiphiles anioniques éventuellement présents dans la composition selon l'invention peuvent notamment être choisis parmi : - - les citrates d'alkyléther, - - les alkényl succinates alkoxylés, - - les alkényl succinates de glucose alkoxylés, - - les alkényl succinates de méthylglucose alkoxylés, - - les sels alcalins du dicétyl- et du dimyristylphosphate ; - - les sels alcalins du cholestérol sulfate ; - - les sels alcalins du cholestérol phosphate ; - - les lipoaminoacides et leurs sels tels que les acylglutamates mono- et di-sodiques comme le sel disodique de l'acide N-stéaroyl L-glutamique commercialisé sous la dénomination Acylglutamate HS21 par la société AJINOMOTO ; - - les sels de sodium de l'acide phosphatidique ; - - les phospholipides - - les dérivés alkylsulfoniques ou alkyléthersulfoniques.

Les citrates d'alkyléther utilisables comme lipides amphiphiles anioniques dans la composition selon l'invention peuvent être choisis notamment dans le groupe comprenant les monoesters, diesters ou triesters formés par l'acide citrique et au moins un alcool gras oxyéthyléné, comportant une chaîne alkyle linéaire ou ramifiée, saturée ou non saturée, ayant de 8 à 22 atomes de carbone, et comportant de 3 à 9 groupes éthoxylés, et leurs mélanges. On peut en effet utiliser un mélange d'un ou plusieurs de ces citrates dans la composition de l'invention.

Ces citrates peuvent être par exemple choisis parmi les mono-, di- et tri-esters d'acide citrique et d'alcoollaurique éthoxylé, comportant de 3 à 9 groupes éthoxylés, commercialisés par la société Witco sous la dénomination Witconol EC, en particulier le Witconol EC 2129 qui est majoritairement un dilaureth-9 citrate, et le Witconol EC 3129 qui est majoritairement un trilaureth-9 citrate.

Les citrates d'alkyléther, utilisés comme tensioactifs sont de préférence employés sous forme neutralisée à un pH d'environ 7, l'agent de neutralisation étant choisi parmi les bases inorganiques telles que la soude, la potasse, l'ammoniac, et les bases organiques telles que la mono-, di- et tri-éthanolamine, l'aminométhylpropanediol-1,3, la N-méthylglucamine, les acides aminés basiques comme l'arginine et la lysine, et leurs mélanges.

Les alkényl succinates utilisables comme lipides amphiphiles anioniques dans la composition de l'invention sont notamment des dérivés éthoxylés et/ou propoxylés et ils sont de préférence choisis parmi les composés de formules (IX) ou (X) :

HOOC-(HR)C-CH₂-COO-E (IX)

HOOC-(HR)C-CH₂-COO-E-O-CO-CH₂-C(HR')-COOH (X)

dans lesquelles :
- les radicaux R et R' sont choisis parmi les radicaux alkényle, linéaires ou ramifiés, comportant de 6 à 22 atomes de carbone,
- E est choisi parmi les chaînes oxyéthylénées de formule (C₂H₄O)ₙ dans laquelle n va de 2 à 100, les chaînes oxypropylénées de formule (C₃H₆O)ₙ dans laquelle n' va de 2 à 100, les copolymères statistiques ou séquencés comprenant des chaînes oxyéthylénées de formule (C₂H₄O)ₙ et des chaînes oxypropylénées de formule (C₃H₆O)_{n'} telles que la somme de n et n' va de 2 à 100, les groupements glucoses oxyéthylénés et/ou oxypropylénés comportant en moyenne de 4 à 100 motifs oxyéthylénés et/ou oxypropylénés répartis sur l'ensemble des fonctions hydroxyle, les groupements méthyl glucoses oxyéthylénés et/ou oxypropylénés comportant en moyenne de 4 à 100 motifs oxyéthylénées et/ou oxypropylénés répartis sur l'ensemble des fonctions hydroxyle.

Dans les formules (IX) et (X), n et n' sont des valeurs moyennes et ne sont donc pas forcément des entiers. On choisit avantageusement pour n une valseur allant de 5 à 60 et encore plus préférentiellement de 10 à 30.

Avantageusement, le radical R et/ou R' est choisi parmi les radicaux alkényle linéaires comportant de 8 à 22 et de préférence de 14 à 22 atomes de carbone. Il peut s'agir par exemple du radical hexadécényl comportant 16 atomes de carbone ou du radical octadécényl comportant 18 atomes de carbone.

Les composés de formules (IX) et (X) décrits ci-dessus dans lesquels E est choisi parmi les chaînes oxyéthylénées, les chaînes oxypropylénées et les copolymères comprenant des chaînes oxyéthylénées et des chaînes oxypropylénées, peuvent être préparés conformément à la description qui est donnée dans les documents WO-A-94/00508, EP-A-107199 et GB-A-2131820 incorporés ici pour référence.

La fonction acide -COOH des lipides amphiphiles anioniques de formules (IX) et (X) se trouve en général dans la composition de l'invention, sous forme neutralisée par un agent de neutralisation, l'agent de neutralisation étant choisi par exemple parmi les bases inorganiques telles que la soude, la potasse, l'ammoniac, et les bases organiques telles que la mono-, di- et tri-éthanolamine, l'aminométhylpropanediol-1,3, la N-méthyl-glucamine, les acides aminés basiques comme l'arginine et la lysine, et leurs mélanges.

A titre d'exemple de lipide amphiphile anionique utilisable dans la composition de l'invention, on peut citer l'hexadécényl succinate 18 OE (composé de formule 1 avec R=hexadécényl, E=(C₂H₄O)ₙ, n=18), l'hexadécényl succinate 45 OE (composé de formule 1 avec R=hexadécényl, E=(C₂H₄O)ₙ, n=45), le dihexadécényl succinate 18 OE (composé de formule II avec R=R'=hexadécényl, E=(C₂H₄O)ₙ, n=18), le dihexadécényl succinate de glucose 10 OE (composé de formule II avec R=R'=hexadécényl, E= glucose oxyéthyléné comportant 10 groupes oxyéthylénés), le dihexadécényl succinate de glucose 20 OE (composé de formule II avec R=R'=hexadécényl, E=glucose oxyéthyléné comportant 20 groupes oxyéthylénés), le dioctadécényl-succinate de méthyl glucose 20 OE (composé de formule II avec R=R'=octadécényl, E= méthyl glucose oxyéthyléné comportant 20 groupes oxyéthylénés), et leurs mélanges.

Le ou les lipides amphiphiles cationiques et/ou anioniques éventuellement présents dans la composition selon l'invention peuvent représenter de 0,1 à 15%, de préférence de 0,2 à 5% en poids du poids total de la composition.

La composition cosmétique selon l'invention peut également comprendre un ou plusieurs polymères.

Le ou les polymères peuvent être d'origine naturelle, végétale ou minérale, et/ou de synthèse.

Par polymère on entend au sens de la présente invention un composé comportant la répétition d'au moins deux unités issues d'au moins un composé appelé monomère. Ceci inclue donc les oligomères avec un nombre de répétition allant de 2 à 10.

Les polymères d'origine naturelle peuvent être choisis parmi les pectines, les celluloses, les alginates, le galactoarabinane, la gomme adragante, les amidons et le saccharose.

Les polymères d'origine végétale et modifiés par voie de synthèse peuvent être choisis par exemple parmi les dérivés d'amidon, tels que carboxyméthylamidon et le phosphate de diamidon, et les dérivés de cellulose tels que l'hydroxyéthylcellulose et la carboxyméthylcellulose.

Les polymères peuvent être choisis parmi les polymères cationiques, anioniques, amphotères et non ioniques.

De préférence les polymères ioniques sont de nature cationique.

Par « polymère cationique », on entend au sens de la présente invention, tout polymère comprenant des groupements cationiques et/ou des groupements ionisables en groupements cationiques.

Les polymères cationiques utilisables dans la composition cosmétique selon l'invention sont de préférence choisis parmi les polymères comportant des groupements amine primaire, secondaire, tertiaire et/ou quaternaire faisant partie de la chaîne polymère ou directement reliés à celle-ci, et ayant une masse moléculaire moyenne en nombre comprise entre 500 et environ 5 000 000, et de préférence entre 1 000 et 3 000 000.

Parmi ces polymères, on peut citer plus particulièrement les polymères cationiques suivants :
(1) les homopolymères ou copolymères d'esters ou d'amides acryliques ou méthacryliques, à fonctions aminées, comportant au moins un des motifs de formules suivantes : 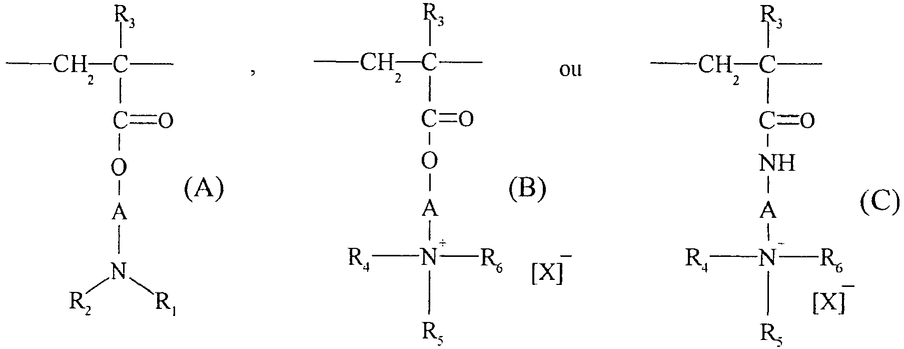 dans lesquelles:
   R₁ et R₂, identiques ou différents, représentent chacun un atome d'hydrogène ou un groupe alkyle ayant de 1 à 6 atomes de carbone ;
   R₃ désigne un atome d'hydrogène ou un groupe CH₃ ;
   A est un groupe alkyle linéaire ou ramifié, comportant de 1 à 6 atomes de carbone ou un groupe hydroxyalkyle comportant de 1 à 4 atomes de carbone ;
   R₄, R₅, R₆, identiques ou différents, représentent un groupe alkyle ayant de 1 à 18 atomes de carbone ou un groupe benzyle ;
   X désigne un anion méthosulfate ou un halogénure tel que chlorure ou bromure.

   Les copolymères de la famille (1) contiennent en outre un ou plusieurs motifs dérivant de comonomères pouvant être choisis dans la famille des acrylamides, méthacrylamides, diacétone-acrylamides, acrylamides et méthacrylamides substitués sur l'azote par des groupes alkyle inférieur (C₁₋₄), des groupes dérivés des acides acryliques ou méthacryliques ou de leurs esters, de vinyllactames tels que la vinylpyrrolidone ou le vinylcaprolactame, d'esters vinyliques.
   Ainsi, parmi ces copolymères de la famille (1), on peut citer :
   - les copolymères d'acrylamide et de méthacrylate de diméthylaminoéthyle quaternisés au sulfate de diméthyle ou avec un halogénure de diméthyle, tels que celui vendu sous la dénomination HERCOFLOC^{®} par la société HERCULES,
   - les copolymères d'acrylamide et de chlorure de méthacryloyloxyéthyltriméthylammonium décrits, par exemple, dans la demande de brevet EP-A-080976 et vendus sous la dénomination BINA QUAT P 100 par la société CIBA GEIGY,
   - les copolymères d'acrylamide et de méthosulfate de méthacryloyloxyéthyltriméthylammonium, tels que celui vendu sous la dénomination RETEN par la société HERCULES,
   - les copolymères vinylpyrrolidone/acrylate ou méthacrylate de dialkylaminoalkyle quaternisés ou non, tels que les produits vendus sous la dénomination "GAFQUAT^{®}" par la société ISP comme, par exemple, "GAFQUAT^{®} 734" ou "GAFQUAT^{®} 755", ou bien les produits dénommés "COPOLYMER^{®} 845, 958 et 937". Ces polymères sont décrits en détail dans les brevets français Nos 2 077 143 et 2 393 573,
   - les terpolymères méthacrylate de diméthylaminoéthyle/ vinylcaprolactame/vinylpyrrolidone tels que le produit commercialisé sous la dénomination GAFFIX^{®} VC 713 par la société ISP, et
   - les copolymères vinylpyrrolidone/méthacrylamide de diméthylaminopropyle quaternisé tels que notamment le produit commercialisé sous la dénomination "GAFQUAT^{®} HS 100" par la société ISP ;
   - les polymères réticulés de sels de méthacryloyloxyalkyl(C1-C4) trialkyl(C1-C4)ammonium tels que les polymères obtenus par homopolymérisation du diméthylaminoéthylméthacrylate quaternisé par le chlorure de méthyle, ou par copolymérisation de l'acrylamide avec le diméthylaminoéthylméthacrylate quaternisé par le chlorure de méthyle, l'homo ou la copolymérisation étant suivie d'une réticulation par un composé à insaturation oléfinique, en particulier le méthylène bis acrylamide. On peut plus particulièrement utiliser un copolymère réticulé acrylamide/chlorure de méthacryloyloxyéthyl triméthylammonium (20/80 en poids) sous forme de dispersion contenant 50 % en poids dudit copolymère dans de l'huile minérale. Cette dispersion est commercialisée sous le nom de " SALCARE® SC 92 " par la Société CIBA. On peut également utiliser un homopolymère réticulé du chlorure de méthacryloyloxyéthyl triméthylammonium contenant environ 50 % en poids de l'homopolymère dans de l'huile minérale ou dans un ester liquide. Ces dispersions sont commercialisées sous les noms de " SALCARE® SC 95 " et " SALCARE® SC 96 " par la Société CIBA.
(2) les polysaccharides cationiques, et en particulier ceux choisis parmi
   a) les dérivés d'éthers de cellulose comportant des groupements ammonium quaternaire décrits dans le brevet FR 1492597. Ces polymères sont également définis dans le dictionnaire CTFA comme des ammoniums quaternaires d'hydroxyéthyl cellulose ayant réagi avec un époxyde substitué par un groupement triméthylammonium ;
   b) les dérivés de cellulose greffés avec un monomère hydrosoluble comportant un ammonium quaternaire, et décrits notamment dans le brevet US 4 131 576, tels que les hydroayalkylcelluloses, comme les hydroxyméthyl-, hydroxyéthyl- ou hydroxypropyl-celluloses greffées notamment avec un sel de méthacryloyloxyéthyl-triméthylammonium, de méthacrylamidopropyl triméthylammonium, de diméthyl-diallylammonium.
      Les produits commercialisés répondant à cette définition sont plus particulièrement les produits vendus sous la dénomination "CELQUAT L 200" et "CELQUAT H 100" par la Société National Starch.
   c) les polygalactomananes cationiques tels que ceux décrits dans les brevets américains 3 589 578 et 4 031 307 tel que les gommes de guar contenant des groupements cationiques trialkylammonium. De tels produits sont commercialisés notamment sous les dénominations commerciales de JAGUAR C13 S, JAGUAR C 15, JAGUAR C 17 par la société MEYHALL ;
(3) les copolymères quaternaires de vinylpyrrolidone et de vinylimidazole ;
(4) les homo ou copolymères d'halogénures de dialkyldiallylammonium et en particulier les composés proposés par la société NALCO sous les noms de MERQUAT 100, MERQUAT 7SPR ou MERQUAT 550.
(5) les chitosanes ou leurs sels ; les sels utilisables sont en particulier l'acétate, le lactate, le glutamate, le gluconate ou le pyrrolidone-carboxylate de chitosane.

Parmi ces composés, on peut citer le chitosane ayant un taux de désacétylation de 90,5 % en poids vendu sous la dénomination KYTAN BRUT STANDARD par la société ABER TECHNOLOGIES, le pyrrolidone-carboxylate de chitosane commercialisé sous la dénomination KYTAMER^{®} PC par la société AMERCHOL.

Le ou les polymères représentent généralement de 0 à 20%, de préférence de 0,2 à 10% en poids, du poids total de la composition.

La composition selon l'invention comprend un milieu cosmétiquement acceptable.

Ce milieu est de préférence aqueux, c'est-à-dire qu'il comprend soit uniquement de l'eau, soit de l'eau et un ou plusieurs solvants tels que par exemple l'éthanol, le propylène glycol, le butylène glycol, l'isopropanol, le glycérol, les éthers de glycol tel que les alkyl (C1-C4) éther de mono, di- ou tripropylène glycol, le mono, di- ou triéthylène glycol, le dipropylène glycol, et leurs mélanges.

La composition selon l'invention peut en outre comprendre tout additif susceptible d'être utilisé dans le domaine d'application considéré.

Les émulsions de l'invention peuvent contenir des actifs hydrosolubles ou liposolubles, ayant une activité cosmétique ou dermopharmaceutique. Les actifs liposolubles sont dans les globules huileux de l'émulsion, tandis que les actifs hydrosolubles sont dans la phase aqueuse de l'émulsion. On peut citer, à titre d'exemples d'actif, les vitamines et leurs dérivés telles que la vitamine E, l'acétate de vitamine E, la vitamine C et ses esters, les vitamines B, la vitamine A alcool ou rétinol, la vitamine A acide ou acide rétinoïque et ses dérivés, les provitamines telles que le panthénol, le palmitate de vitamine A, la niacinamide, l'ergocalciférol, les anti oxydants, les humectants, les filtres solaires siliconés ou non, des agents conservateurs, des séquestrants, des adoucissants, des colorants, des agents modificateurs de viscosité, des agents modificateurs de mousse, des stabilisateurs de mousse, des agents nacrants, des pigments, des agents hydratants, des agents antipelliculaires, des agents antiséborrhéiques, des protéines, des céramides, des pseudocéramides, des acides gras à chaînes linéaires ou ramifiées en C₁₆-C₄₀ tels que l'acide méthyl-18 eicosanoique, des plastifiants, des hydroxyacides, des électrolytes, et des parfums.

Les nanoémulsions de l'invention peuvent être obtenues par un procédé, caractérisé par le fait qu'on mélange la phase aqueuse et la phase huileuse, sous agitation vive, à une température ambiante inférieure à 45°C puis qu'on effectue une homogénéisation haute pression à une pression supérieure à 5.10⁷ Pa et de préférence allant de 6.10⁷ à 18.10⁷ Pa. Un tel procédé permet de réaliser, à température ambiante, des nanoémulsions compatibles avec des composés actifs thermosensibles, et pouvant contenir des quantités importantes d'huiles et notamment des parfums qui renferment des corps gras, sans les dénaturer.

On peut aussi les obtenir par un procédé par dilution comme décrit dans la demande FR2847831.

L'invention a encore pour objet l'utilisation de la composition telle que définie précédemment pour le conditionnement des cheveux.

L'invention est illustrée par les exemples qui suivent.

### Exempte 1

On prépare différentes compositions sous forme de nanoémulsion. Les formulations sont données dans le tableau 1. Les teneurs sont exprimées en g de produit tel quel pour 100g de composition.

La composition 1 n'est pas une composition selon l'invention. La composition 2 est une composition selon l'invention.

**Tableau 1**

| | **1** | **2** |
|---|---|---|
| Cire liquide de Jojoba | 6 | 6 |
| Huile d'avocat | 5 | 5 |
| Cyclopentadiméthylsiloxane (DOW CORNING) | 3 | |
| Mélange Undécane/tridécane selon l'exemple 2 de WO 2008/155059 | | 3 |
| Poly diméthylsiloxane à groupements aminoéthyl iminopropyle en microémulsion non ionique à 17% dans l'eau (WACKER) | 5 | 5 |
| Alcool éthylique 96 degrés | 17 | 17 |
| Glycérol | 4 | 4 |
| Chlorure de béhényl triméthyl ammonium à 79% (CLARIANT) | 2 | 2 |
| Mono-isostéarate de polyéthylène glycol, (8 OE) (CRODA) | 3 | 3 |
| Parfum | qs | |
| Eau désionisée | Qsp 100 | |

La viscosité des compositions 1 et 2 est mesurée au viscosimètre Brookfield DVII+Pro, à 25°C. La lecture est effectuée à une minute. Les résultats sont donnés en cps, dans le tableau 2.

**Tableau 2**

| Composition | 1 | 2 |
|---|---|---|
| Viscosité en cps | 135 | 8500 |

Les alcanes linéaires volatils confèrent un épaississement plus important que les autres solvants classiquement utilisés tels que le cyctopentadiméthyisitoxane.

### Exemple 2

On prépare différentes compositions sous forme de nanoémulsion. Les formulations sont données dans le tableau 2. Les teneurs sont exprimées en g de produit tel quel pour 100g de composition.

Les compositions 3 et 4 sont des compositions selon l'invention.

**Tableau 3**

| | 3 | 4 |
|---|---|---|
| Cire liquide de Jojoba | 6 | 6 |
| Huile d'avocat stabilisée | 5 | 5 |
| Mélange n-Undécane/n-tridécane selon l'exemple 2 de WO 2008/155059 | 3 | |
| Mélange n-Dodécane/n-tétradécane (VEGELIGHT 1214 de Biosynthis) | | 3 |
| Poly diméthylsiloxane à groupements aminoéthyl iminopropyle en microémulsion non ionique à 17% dans l'eau (WACKER) | 5 | 5 |
| Alcool éthylique 96 degrés | 17 | 17 |
| Glycérol | 4 | 4 |
| Chlorure de béhényl triméthyl ammonium à 79% (CLARIANT) | 2 | 2 |
| Mono-isostéarate de polyéthylène glycol (8 OE) (CRODA) | 2 | 3 |
| Parfum | qs | |
| Eau désionisée | Qsp 100 | |

## Revendications

1. Composition cosmétique sous forme de nanoémulsion huile-dans-eau, **caractérisée en ce qu'**elle comprend, dans un milieu cosmétiquement acceptable, un ou plusieurs lipides amphiphiles non-ioniques, de 2 à 15 % en poids par rapport au poids total de la composition d'un ou plusieurs alcanes linéaires volatils, un ou plusieurs lipides amphiphiles cationiques et une ou plusieurs huiles différentes du ou desdits alcanes linéaires volatils, le ou les alcanes linéaires volatils comprenant de 7 à 15 atomes de carbone,
la taille moyenne en nombre des globules de la nanoémulsions étant compris entre 1 et 350 nm.

2. Composition selon la revendication 1 **caractérisée en ce que** le ou les alcanes linéaires volatils sont les alcanes linéaires comprenant de 8 à 14 atomes de carbone, mieux de 11 à 14 atomes de carbone.

3. Composition selon l'une quelconque des revendications précédentes **caractérisée en ce que** le ou les alcanes linéaires volatils sont d'origine végétale.

4. Composition selon l'une quelconque des revendications précédentes **caractérisée en ce que** le ou les alcanes linéaires volatils sont choisis parmi le n- heptane, le n-octane, le n-nonane, le n-décane, le n-undécane, le n-dodécane, le n-tridécane, le n-tétradécane, et leurs mélanges.

5. Composition selon la revendication 4 **caractérisé en ce que** le ou les alcanes linéaires volatils sont choisis parmi le n-nonane, le n-undécane, le n-dodécane, le n-tridécane, le n-tétradécane, et leurs mélanges.

6. Composition selon la revendication 5 **caractérisée en ce que** les alcanes linéaires volatils sont un mélange n-undécane/n-tridécane.

7. Composition selon l'une quelconque des revendications précédentes **caractérisé en ce que** le ou les lipides amphiphiles non-ioniques de l'invention sont choisis parmi :
1/- les tensioactifs siliconés,
2/- les lipides amphiphiles fluides à température inférieure ou égale à 45°C choisis parmi les esters d'au moins un polyol choisi dans le groupe formé par le polyéthylèneglycol comportant de 1 à 60 unités d'oxyde d'éthylène, le sorbitane, le glycérol comportant de 2 à 30 unités d'oxyde d'éthylène, les polyglycérols comportant de 2 à 15 unités de glycérol et d'au moins un acide gras comportant au moins une chaîne alkyle en C₈-C₂₂, saturée ou non saturée, linéaire ou ramifiée,
3/- les esters mixtes d'acide gras en C₈-C₂₂ ou d'alcool gras en C₈-C₂₂, d'acide carboxylique et de glycérol,
4/- les esters d'acide gras et de sucre et les éthers d'alcool gras et de sucre,
5/- les tensioactifs solides à une température inférieure ou égale à 45°C, choisis parmi les esters gras de glycérol, les esters gras de sorbitan et les esters gras de sorbitan oxyéthylénés, les éthers gras éthoxylés et les esters gras éthoxylés, et
6/- les copolymères blocs d'oxyde d'éthylène (A) et d'oxyde de propylène (B).

8. Composition selon la revendication 7 **caractérisée en ce que** le ou les lipides amphiphiles non-ioniques sont choisis parmi :
- l'isostéarate de polyéthylèneglycol (8 moles d'oxyde d'éthylène),
- l'isostéarate de diglycéryle,
- le monolaurate et le monostéarate de polyglycérol comportant 10 unités de glycérol,
- l'oléate de sorbitane,
- l'isostéarate de sorbitane.

9. Composition selon l'une quelconque des revendications précédentes **caractérisé en ce que** le ou les lipides amphiphiles non ioniques représentent de 0,1 à 30%, de préférence de 0,5 à 20%, mieux de 1 à 10%, en poids du poids total de la composition.

10. Composition selon l'une quelconque des revendications précédentes **caractérisé en ce que** l'huile ou les huiles est (sont) choisie(s) parmi les huiles végétales, les huiles animales, les huiles minérales, les huiles synthétiques, les alcools gras liquides, les huiles siliconées et leurs mélanges et de préférence est (sont) choisie(s) parmi les huiles végétales, et plus particulièrement l'huile d'olive et la cire liquide de jojoba ; les huiles siliconées et plus particulièrement les polydiméthylsiloxanes linéaires ou cycliques; et les huiles minérales telle que l'huile de vaseline.

11. Composition selon l'une quelconque des revendications précédentes, **caractérisée en ce que** l'huile ou les huiles sont présentes en une quantité allant de 1 à 40 % en poids, de préférence de 2 à 15 % en poids, par rapport au poids total de la composition.

12. Composition selon l'une quelconque des revendications précédentes, **caractérisée en ce que** le rapport pondéral de la quantité d'huile(s) sur la quantité de lipide(s) amphiphile(s) non-ionique(s) est inférieur ou égal à 7, mieux compris entre 0,5 et 7, et encore plus préférentiellement inférieur ou égal à 5, mieux compris entre 0,5 et 5.

13. Utilisation de la composition telle que définie dans l'une quelconque des revendications précédentes pour le conditionnement des cheveux.

## Patentansprüche

1. Kosmetische Zusammensetzung in Form einer Öl-in-Wasser-Nanoemulsion, **dadurch gekennzeichnet, dass** sie in einem kosmetisch unbedenklichen Medium ein oder mehrere nichtionische amphiphile Lipide, 2 bis 15 Gew.-%, bezogen auf das Gesamtgewicht der Zusammensetzung, eines oder mehrerer flüchtiger linearer Alkane, ein oder mehrere kationische amphiphile Lipide und ein oder mehrere Öle, die von dem flüchtigen linearen Alkan bzw. den flüchtigen linearen Alkanen verschieden ist bzw. sind, wobei das flüchtige lineare Alkan bzw. die flüchtigen linearen Alkane 7 bis 15 Kohlenstoffatome enthalten,
wobei die zahlenmittlere Größe der Nanoemulsionskügelchen zwischen 1 und 350 nm liegt.

2. Zusammensetzung nach Anspruch 1, **dadurch gekennzeichnet, dass** es sich bei dem flüchtigen linearen Alkan bzw. den flüchtigen linearen Alkanen um lineare Alkane mit 8 bis 14 Kohlenstoffatomen und noch besser 11 bis 14 Kohlenstoffatomen handelt.

3. Zusammensetzung nach einem der vorhergehenden Ansprüche, **dadurch gekennzeichnet, dass** das flüchtige lineare Alkan bzw. die flüchtigen linearen Alkane pflanzlichen Ursprungs ist bzw. sind.

4. Zusammensetzung nach einem der vorhergehenden Ansprüche, **dadurch gekennzeichnet, dass** das flüchtige lineare Alkan bzw. die flüchtigen linearen Alkane aus n-Heptan, n-Octan, n-Nonan, n-Decan, n-Undecan, n-Dodecan, n-Tridecan, n-Tetradecan und Mischungen davon ausgewählt ist bzw. sind.

5. Zusammensetzung nach Anspruch 4, **dadurch gekennzeichnet, dass** das flüchtige lineare Alkan bzw. die flüchtigen linearen Alkane aus n-Nonan, n-Undecan, n-Dodecan, n-Tridecan, n-Tetradecan und Mischungen davon ausgewählt ist bzw. sind.

6. Zusammensetzung nach Anspruch 5, **dadurch gekennzeichnet, dass** es sich bei den flüchtigen linearen Alkanen um eine Mischung von n-Undecan und n-Tridecan handelt.

7. Zusammensetzung nach einem der vorhergehenden Ansprüche, **dadurch gekennzeichnet, dass** das nichtionische amphiphile Lipid bzw. die nichtionischen amphiphilen Lipide der Erfindung aus:
1) Silikontensiden,
2) bei einer Temperatur kleiner gleich 45°C fließfähigen amphiphilen Lipiden, ausgewählt aus Estern von mindestens einem Polyol aus der Gruppe bestehend aus Polyethylenglykol mit 1 bis 60 Ethylenoxid-Einheiten, Sorbitan, Glycerin mit 2 bis 30 Ethylenoxid-Einheiten, Polyglycerinen mit 2 bis 15 Glycerin-Einheiten und mindestens einer Fettsäure mit mindestens einer gesättigten oder ungesättigten, linearen oder verzweigten C₈-C₂₂-Alkylkette,
3) gemischten Estern von C₈-C₂₂-Fettsäure oder C₈-C₂₂-Fettalkohol, Carbonsäure und Glycerin,
4) Zuckerfettsäureestern und Zuckerfettalkoholethern,
5) bei einer Temperatur kleiner gleich 45°C festen Tensiden, ausgewählt aus Glycerinfettsäureestern, Sorbitanfettsäureestern und oxyethylierten Sorbitanfettsäureestern, ethoxylierten Fettethern und ethoxylierten Fettestern, und
6) Blockcopolymeren von Ethylenoxid (A) und Propylenoxid (B).
ausgewählt ist bzw. sind.

8. Zusammensetzung nach Anspruch 7, **dadurch gekennzeichnet, dass** das nichtionische amphiphile Lipid bzw. die nichtionischen amphiphilen Lipide der Erfindung aus:
- Polyethylenglykolisostearat (8 mol Ethylenoxid),
- Diglycerylisostearat,
- Polyglycerinmonolaurat und -monostearat mit zehn Glycerin-Einheiten,
- Sorbitanoleat,
- Sorbitanisostearat
ausgewählt ist bzw. sind.

9. Zusammensetzung nach einem der vorhergehenden Ansprüche, **dadurch gekennzeichnet, dass** das nichtionische amphiphile Lipid bzw. die nichtionischen amphiphilen Lipide 0,1 bis 30 Gew.-%, vorzugsweise 0,5 bis 20 Gew.-% und noch besser 1 bis 10 Gew.-% des Gesamtgewichts der Zusammensetzung ausmacht bzw. ausmachen.

10. Zusammensetzung nach einem der vorhergehenden Ansprüche, **dadurch gekennzeichnet, dass** das Öl bzw. die Öle aus pflanzlichen Ölen, tierischen Ölen, Mineralölen, synthetischen Ölen, flüssigen Fettalkoholen, Silikonölen und Mischungen davon ausgewählt ist bzw. sind und vorzugsweise aus pflanzlichen Ölen und spezieller Olivenöl und flüssigem Jojobawachs, Silikonölen und spezieller linearen oder cyclischen Polydimethylsiloxanen und Mineralölen wie Vaselineöl ausgewählt ist bzw. sind.

11. Zusammensetzung nach einem der vorhergehenden Ansprüche, **dadurch gekennzeichnet, dass** das Öl bzw. die Öle in einer Menge im Bereich von 1 bis 40 Gew.-% und vorzugsweise 2 bis 15 Gew.-%, bezogen auf das Gesamtgewicht der Zusammensetzung, vorliegen.

12. Zusammensetzung nach einem der vorhergehenden Ansprüche, **dadurch gekennzeichnet, dass** das Gewichtsverhältnis der Menge an Öl bzw. Ölen zur Menge an nichtionischem amphiphilem Lipid bzw. nichtionischen amphiphilen Lipiden kleiner gleich 7 ist, noch besser zwischen 0,5 und 7 liegt, noch weiter bevorzugt kleiner gleich 5 ist und noch besser zwischen 0,5 und 5 liegt.

13. Verwendung der Zusammensetzung gemäß einem der vorhergehenden Ansprüche zur Konditionierung der Haare.

## Claims

1. Cosmetic composition in the form of an oil-in-water nanoemulsion, **characterized in that** it comprises, in a cosmetically acceptable medium, one or more nonionic amphiphilic lipids, from 2% to 15% by weight relative to the total weight of the composition of one or more volatile linear alkanes, one or more cationic amphiphilic lipids and one or more oils other than the said volatile linear alkane(s), the volatile linear alkane or alkanes comprising from 7 to 15 carbon atoms,
the number-average size of the globules of the nanoemulsion being between 1 and 350 nm.

2. Composition according to Claim 1, **characterized in that** the volatile linear alkane(s) are linear alkanes comprising from 8 to 14 carbon atoms, better from 11 to 14 carbon atoms.

3. Composition according to either of the preceding claims, **characterized in that** the volatile linear alkane(s) are of plant origin.

4. Composition according to any one of the preceding claims, **characterized in that** the volatile linear alkane(s) are chosen from n-heptane, n-octane, n-nonane, n-decane, n-undecane, n-dodecane, n-tridecane and n-tetradecane, and mixtures thereof.

5. Composition according to Claim 4, **characterized in that** the volatile linear alkane(s) are chosen from n-nonane, n-undecane, n-dodecane, n-tridecane and n-tetradecane, and mixtures thereof.

6. Composition according to Claim 5, **characterized in that** the volatile linear alkanes are an n-undecane/n-tridecane mixture.

7. Composition according to any one of the preceding claims, **characterized in that** the nonionic amphiphilic lipid(s) of the invention are chosen from:
1) silicone surfactants,
2) amphiphilic lipids that are fluid at a temperature of less than or equal to 45°C, chosen from the esters of at least one polyol chosen from the group formed by polyethylene glycol comprising from 1 to 60 ethylene oxide units, sorbitan, glycerol comprising from 2 to 30 ethylene oxide units, and polyglycerols comprising from 2 to 15 glycerol units, and of at least one fatty acid comprising at least one saturated or unsaturated, linear or branched C₈-C₂₂ alkyl chain,
3) mixed esters of C₈-C₂₂ fatty acid or of C₈-C₂₂ fatty alcohol, of carboxylic acid and of glycerol,
4) fatty acid esters of sugars and fatty alkyl ethers of sugars,
5) surfactants that are solid at a temperature of less than or equal to 45°C, chosen from fatty esters of glycerol, fatty esters of sorbitan and oxyethylenated fatty esters of sorbitan, ethoxylated fatty ethers and ethoxylated fatty esters, and
6) block copolymers of ethylene oxide (A) and of propylene oxide (B).

8. Composition according to Claim 7, **characterized in that** the nonionic amphiphilic lipid(s) are chosen from:
- polyethylene glycol isostearate (8 mol of ethylene oxide),
- diglyceryl isostearate,
- polyglyceryl monolaurate and monostearate comprising 10 glycerol units,
- sorbitan oleate,
- sorbitan isostearate.

9. Composition according to any one of the preceding claims, **characterized in that** the nonionic amphiphilic lipid(s) represent from 0.1% to 30%, preferably from 0.5% to 20% and better still from 1% to 10% by weight relative to the total weight of the composition.

10. Composition according to any one of the preceding claims, **characterized in that** the oil(s) are chosen from plant oils, animal oils, mineral oils, synthetic oils, liquid fatty alcohols and silicone oils, and mixtures thereof, and are preferably chosen from plant oils, and more particularly olive oil and liquid jojoba wax; silicone oils and more particularly linear or cyclic polydimethylsiloxanes; and mineral oils such as liquid petroleum jelly.

11. Composition according to any one of the preceding claims, **characterized in that** the oil(s) are present in an amount ranging from 1% to 40% by weight and preferably from 2% to 15% by weight relative to the total weight of the composition.

12. Composition according to any one of the preceding claims, **characterized in that** the weight ratio of the amount of oil(s) to the amount of nonionic amphiphilic lipid(s) is less than or equal to 7, better still between 0.5 and 7, more preferentially less than or equal to 5 and better still between 0.5 and 5.

13. Use of the composition as defined in any one of the preceding claims, for conditioning the hair
